# EUROPÄISCHE PATENTANMELDUNG

(11) **EP 0 844 480 A2**
(43) Veröffentlichungstag der Anmeldung: **27.05.1998**
(21) Anmeldenummer: 97116939.6
(22) Anmeldetag: 30.09.1997
(51) Int. Cl.: G01N 33/18, G01N 31/22

(54) **Vorrichtung und Verfahren zum Anzeigen der Erschöpfung eines Entkarbonisierungs- und/oder Enthärtungsmittels für Wasser**

(30) Priorität: 20.11.1996 DE 19647971
(71) Anmelder: Brita Wasser-Filter-Systeme GmbH, D-65232 Taunusstein-Neuhof (DE)
(72) Erfinder: Sauerbier, Knut, 65658 Fürth (DE); Lang, Uwe, Dr., 65232 Taunusstein (DE)
(74) Vertreter: Weber, Dieter, Dr.

(57) **Zusammenfassung**

Eine Vorrichtung zum Anzeigen der Erschöpfung eines Entkarbonisierungs- und/oder Enthärtungsmittels für Wasser besitzt eine saugfähige Matrix, in einer Indikatorlinie in der Matrix angeordneten lösemittellöslichen Wasserhärte-Indikator, der entlang der Indikatorlinie in derart zunehmend veränderter Weise enthalten ist, daß die Umschlaggrenze des Indikators in Abhängigkeit von der Härte eines mit dem Wasserhärte-Indikator in Berührung gebrachten Wassers an unterschiedlichen Stellen auf der Indikatorlinie liegt, und entweder eine im Abstand von der Indikatorlinie auf der Matrix angebrachte, einer Skaleneinteilung zugeordnete Zeitlinie oder eine Ansaugkante der Matrix, wobei die Zeitlinie bzw. Ansaugkante dem Punkt der Indikatorlinie, wo die Umschlaggrenze der größten Wasserhärte entspricht, am nächsten liegt und von dem Punkt der Indikatorlinie, wo die Umschlaggrenze der kleinsten Wasserhärte entspricht, am weitesten enfernt liegt, oder umgekehrt. Ein Verfahren verwendet diese Vorrichtung.

## Beschreibung

Für die Entkarbonisierung und/oder Enthärtung von Wasser sind Filtermaterialien bekannt, die gewöhnlich in der Hauptsache aus Ionenaustauschern und/oder Aktivkohle bestehen. Für die Verwendung solcher Entkarbonisierungs- und/oder Enthärtungsmaterialien sind unterschiedliche Geräte bekannt, die je nach Bauart und Größen für Haushaltszwecke oder gewerbliche Zwecke bestimmt sind.

In allen Fällen tritt bei solchen Filtermaterialien nach einer bestimmten Zeit eine Erschöpfung der Wirksamkeit ein. Zu diesem Zeitpunkt müssen die Entkarbonisierungs- und/oder Enthärtungsmittel dann regeneriert werden, was in größeren gewerblich genutzten Anlagen in der Anlage selbst geschehen kann, bei Haushaltzwasserfiltern aber meist durch Austausch einer Patrone erfolgt, die das Entkarbonisierungsmittel enthält.

Es sind Farbindikatoren zur Bestimmung der Karbonathärte oder Gesamthärte etwa in der Form von Einmalmeßstäbchen bekannt. Auch wasserlösliche Farbindikatoren für diesen Zweck gehören zum Stand der Technik. Mit solchen Indikatoren oder Meßstäbchen kann bestimmt werden, ob Entkarbonisierungs- und/oder Enthärtungsmittel noch ausreichend wirksam oder bereits erschöpft sind, doch muß zu einer solchen Ermittlung laufend in bestimmten Abständen der Indikatortest durchgeführt werden, was insbesondere bei Haushaltsfiltern nicht realisierbar ist. Es ist auch bereits bekannt, Wasserfiltergeräte mit einer Zeitanzeige zu versehen, mit Hilfe derer der Benutzer bestimmen kann, wie lange das Entkarbonisierungs- und/oder Enthärtungsmittel bereits in Benutzung ist.

Derartige Anzeigeeinrichtungen geben den Erschöpfungszustand des Mittels jedoch nur sehr pauschal wieder, da der Zeitraum bis zur Erschöpfung wesentlich von dem Härtegrad des zu behandelnden Wassers abhängt und bei Behandlung von weicherem Wasser die Erschöpfung des Mittels erheblich später als bei härterem Wasser eintritt. Da Wasserfilter in vielen Regionen mit sehr unterschiedlichen Wasserhärten eingesetzt werden, kann eine Anzeige der Benutzungszeit den Erschöpfungszeitpunkt nur ungenau angeben, so daß gegen Ende der Benutzungszeit eines Entkarbonisierungs- und/oder Enthärtungsmittels vor der Regenerierung die Gefahr besteht, daß das filtrierte Wasser nur ungenügend entkarbonisiert oder enthärtet wird.

Die der Erfindung zugrundeliegende Aufgabe bestand somit darin, eine Vorrichtung zum Anzeigen der Erschöpfung eines Entkarbonisierungs- und/oder Enthärtungsmittels für Wasser zu bekommen, die nicht nur die Verwendungszeit, sondern auch gleichzeitig den Härtegrad des zu behandelnden Wassers berücksichtigt und daher die Erschöpfung des Mittels genauer als bisher bekannte Vorrichtungen anzeigt.

Diese Aufgabe wird erfindungsgemäß einerseits durch eine Vorrichtung gelöst, die gekennzeichnet ist durch eine saugfähige Matrix, in einer Indikatorlinie in der Matrix angeordneten lösemittellöslichen Wasserhärte-Indikator, der entlang der Indikatorlinie in derart zunehmend veränderter Weise enthalten ist, daß die Umschlaggrenze des Indikators in Abhängigkeit von der Härte eines mit dem Wasserhärte-Indikator in Berührung gebrachten Wassers an unterschiedlichen Stellen auf der Indikatorlinie liegt, und eine im Abstand von der Indikatorlinie auf der Matrix angebrachte, einer Skaleneinteilung zugeordnete Zeitlinie, wobei die Zeitlinie dem Punkt der Indikatorlinie, wo die Umschlaggrenze der größten Wasserhärte entspricht, am nächsten liegt und von dem Punkt der Indikatorlinie, wo die Umschlaggrenze der kleinsten Wasserhärte entspricht, am weitesten entfernt liegt, oder umgekehrt.

Andererseits wird die Aufgabe auch durch eine Vorrichtung gelöst, die gekennzeichnet ist durch eine saugfähige Matrix mit einer Ansaugkante, in einer Indikatorlinie in der Matrix angeordneten matrixgebundenen Wasserhärte-Indikator, der entlang der Indikatorlinie in derart zunehmend veränderter Weise enthalten ist, daß die Umschlaggrenze des Indikators in Abhängigkeit von der Härte eines mit dem Wasserhärte-Indikator in Berührung gebrachten Wassers an unterschiedlichen Stellen auf der Indikatorlinie liegt, wobei die Ansaugkante dem Punkt der Indikatorlinie, wo die Umschlaggrenze der größten Wasserhärte entspricht, am nächsten liegt und von dem Punkt der Indikatorlinie, wo die Umschlaggrenze der kleinsten Wasserhärte entspricht, am weitesten entfernt liegt, oder umgekehrt.

Beide Ausführungsformen der erfindungsgemäßen Vorrichtung machen in Umkehr der Merkmale vom gleichen Lösungsprinzip Gebrauch. Bei der ersten Ausführungsform enthält die Matrix lösemittellöslichen, wie insbesondere wasserlöslichen, Indikator, der durch Eintauchen einer Ansaugkante der Matrix in das Lösemittel zu der Zeitlinie diffundiert und den Erschöpfungszeitpunkt anzeigt, wenn die Umschlaggrenze des Indikators die Zeitlinie erreicht, während bei der zweiten Ausführungsform der Indikator nicht löslich, sondern matrixgebunden im Abstand von der Ansaugkante der Matrix angeordnet ist, wobei eine Teilmenge des zu entkarbonisierenden oder zu enthärtenden Wassers bis zu der Indikatorlinie diffundiert und dort an der für die spezielle Wasserhärte des zu behandelnden Wassers relevanten Stelle der Indikatorlinie den Indikatorumschlag bewirkt und damit die Erschöpfung des Mittels anzeigt.

Die erste Ausführungsform hat den Vorteil, daß man den Erschöpfungsverlauf des Mittels beobachten kann, während die zweite Ausführungsform den Vorteil hat, kein zusätzliches Lösemittel zu erfordern. Auch bei der zweiten Ausführungsform läßt sich der Erschöpfungsverlauf beobachten, wenn bei dieser Ausführungsform zwischen der Ansaugkante und der Indikatorlinie weiterer Wasserhärte-Indikator in der Form eines Indikatorfeldes in der Matrix angeordnet ist, dessen Beschaffenheit von der Ansaugkante zur Indikatorlinie im wesentlichen unverändert ist, sich aber senkrecht hierzu in seinem Verlauf zunehmend verändert.

Die erfindungsgemäßen Vorrichtungen können sowohl für Mittel zur Verminderung der Karbonathärte, die durch Calciumhydrogenkarbonat und Magnesiumhydrogenkarbonat bewirkt wird und durch Austausch der Kationen von Calcium und Magnesium gegen Wasserstoff vermindert wird, als auch für die Verminderung der Nichtkarbonathärte oder Permanenthärte, die beispielsweise durch Calciumchlorid oder Calciumsulfat hervorgerufen wird und durch Austausch von Calcium- gegen Natriumkationen vermindert wird, als auch zur Verminderung der Gesamthärte verwendet werden. Indikatoren für diese unterschiedlichen Entkarbonisierungen und/oder Enthärtungen sind nach dem Stand der Technik bekannt.

Wenn in diesem Zusammenhang von einem Umschlag oder einer Umschlaggrenze des Indikators die Rede ist, so ist damit jede feststellbare Veränderung eines Indikators gemeint. Im Regelfall wird man solche Indikatoren bevorzugen, die bei einer bestimmten Härte des Wassers einen Farbumschlag ergeben, da dieser direkt visuell bestimmbar ist. Die Erfindung soll aber auch solche Indikatoren einschließen, deren Veränderung oder Umschlag nur mit bestimmten Hilfsmitteln, wie UV-Bestrahlung oder dergleichen, feststellbar ist.

Als Matrixmaterial kommt für die Erfindung jedes saugfähige Material in Betracht, das in der Lage ist, durch Kapillarkräfte Wasser oder ein anderes Lösemittel für den Indikator durch Diffusion bei der ersten Ausführungsform bis mindestens zur Zeitlinie, bei der zweiten Ausführungsform bis mindestens zur Indikatorlinie zu transportieren. Beispiele solcher Matrixmaterialien sind Cellulosefaservliese, Kieselgelkörper und -ansammlungen, Solgele oder andere bekannte saugfähige Matrixmaterialien. Das Matrixmaterial ist nicht nur nach seiner Diffusionseigenschaft, sondern auch nach der Diffusionsgeschwindigkeit auszuwählen. Für Einmaltests ist es erwünscht, Matrixmaterialien zu verwenden, in denen das Wasser oder Lösemittel rasch zur Ziellinie diffundiert, während beispielsweise bei Anbringung der Vorrichtung an einer Wasserfilterpatrone ein Matrixmaterial erwünscht sein kann, in welchem Wasser oder Lösemittel nur langsam bis zur Ziellinie diffundiert, wie beispielsweise ein an sich bekanntes Solgel. Die Matrix kann unterschiedliche Form haben, liegt aber vorzugsweise in Form einer ebenen oder gekrümmten Platte mit einer Ansaugkante vor.

Wenn in Verbindung mit den verschiedenen Ausführungsformen der Erfindung davon die Rede ist, daß der Indikator entlang der Indikatorlinie, zum Beispiel von deren einem zum anderen Ende, in zunehmend veränderter Weise enthalten sein soll, so kann diese Veränderung innerhalb der Indikatorlinie unterschiedlicher Natur sein. Beispielsweise kann die Indikatorkonzentration vom einen Ende der Indikatorlinie zu ihrem anderen Ende kontinuierlich oder intermittierend zunehmen oder abnehmen, oder es können entlang der Indikatorlinie unterschiedliche Indikatoren angeordnet sein, die jeweils bei einem bestimmten Härtegrad des mit ihnen in Berührung tretenden Wassers umschlagen. Die Indikatorlinie kann eine zusammenhängende linienförmige Aufbringung des Indikators auf dem Matrixmaterial sein oder sich aus einzelnen Indikatorfeldern zusammensetzen, die im Abstand voneinander entlang der Indikatorlinie auf dem Matrixmaterial aufgebracht sind. In gleicher Weise kann bei der zweiten Ausführungsform das Indikatorfeld eine zusammenhänge Fläche mit in einer Richtung kontinuierlich oder intermittierend zunehmender Veränderung oder aber eine Ansammlung einzelner Indikatorflecken bzw. voneinander getrennter mit Indikator versehener Bereiche des Matrixmaterials sein.

Die Indikatorlinie wie auch die Zeitlinie und Ansaugkante des Matrixmaterials können geradlinig, gekrümmt oder stufenweise ausgebildet sein oder ein Minimum oder Maximum im Kurvenverlauf haben, wobei es bevorzugt ist, geradlinige Indikatorlinien, Zeitlinien und Ansaugkanten zu haben. Bevorzugt ist, daß bei der ersten Ausführungsform der Abstand zwischen der Indikatorlinie und der Zeitlinie, bei der zweiten Ausführungsform der Abstand zwischen der Ansaugkante und der Indikatorlinie derart ist, daß der Abstand am einen Ende dieser Linien größer als am anderen Ende ist, wobei auf der Indikatorlinie jeweils das eine Ende bei der größten Wasserhärte und das andere Ende bei der kleinsten Wassserhärte umschlägt.

Die wenigstens bei der ersten Ausführungsform vorgesehene Skaleneinteilung kann der Zeitlinie in unterschiedlicher Weise zugeordnet sein und beliebige Aufteilung besitzen. So kann die Skaleneinteilung direkt auf der Zeitlinie angebracht oder seitlich oder darüber vorgesehen sein. Wesentlich ist lediglich, daß man bei Erreichen der Zeitlinie durch die Indikatorumschlaggrenze automatisch und zwangsläufig die Erschöpfungszeit bei Behandlung des speziellen Wassers mit einer bestimmten Wasserhärte angezeigt bekommt. Bei der zweiten Ausführungsform ist überhaupt keine Skaleneinteilung erforderlich, da hier der absolute Erschöpfungszeitpunkt angezeigt wird. Selbstverständlich kann auch hier eine Skaleneinteilung vorgesehen sein.

Wenn im Zusammenhang mit der Erfindung von einer Umschlaggrenze des Indikators in Abhängigkeit von der Härte des Wassers die Rede ist, so bedeutet dies, daß der entlang der Indikatorlinie in kontinuierlich oder diskontinuierlich veränderter Beschaffenheit aufgebrachte Indikator bei Berührung mit Wasser einer bestimmten Härte bis zu einem bestimmten Punkt umschlägt, d. h., daß der Indikator oder die Indikatoren entweder für alle geringeren Härtegrade oder für alle höheren Härtegrade bis zu dem Härtegrad des mit ihnen in Berührung gebrachten Wassers umschlagen. Bei der Verwendung von Farbindikatoren bekommt man somit auf der Indikatorlinie in Abhängigkeit von der Wasserhärte des zu behandelnden Wassers eine Grenze zwischen zwei Farben (wie beispielsweise rot und grün), die sich nicht mehr verschiebt und im Falle der ersten Ausführungsform beim Auftreffen auf die Zeitlinie den Erschöpfungszeitraum angibt und bei der zweiten Ausführungsform der Erfindung bei der örtlichen Stabilisierung direkt den Erschöpfungszustand anzeigt, bei dem eine Regenerierung des Entkarbonisierungs- und/oder Enthärtungsmittels vorgenommen werden muß.

Bei der ersten Ausführungsform nach der Erfindung können nach einer Variante der Indikator oder die Indikatoren entlang der Indikatorlinie gemeinsam und gleichzeitig zu der Zeitlinie hin diffundieren. Bei Aufbringung unterschiedlicher Indikatoren entlang der Indikatorlinie können die Indikatoren aber auch so ausgewählt werden, daß nur oder bevorzugt jener Indikator zur Zeitlinie diffundiert, der entweder umgeschlagen oder nicht umgeschlagen ist.

Nachfolgend ist die Wirkungsweise und Anwendung verschiedener Varianten der erfindungsgemäßen Vorrichtungen unter Verwendung des erfindungsgemäßen Verfahrens beschrieben.

In der Zeichnung bedeuten
Figuren 1 bis 5
   schematische Darstellungen von fünf Varianten der erfindungsgemäßen Vorrichtung, die nachfolgend erläutert werden.

In jeder dieser Figuren ist in der linken Hälfte eine plattenartige Matrix vor der Verwendung und in der rechten Hälfte die gleiche Plattenmatrix zum Zeitpunkt der Anzeige der Erschöpfung wiedergegeben, wobei jeweils am unteren Ende der Plattenmatrix schematisch eine Schale für Flüssigkeit angedeutet ist, welche in die Matrix diffundieren soll.

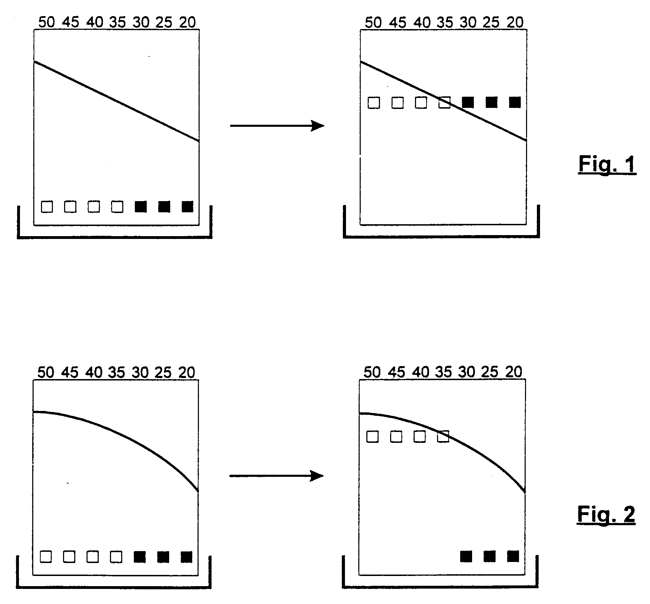

### Erste Variante (Fig. 1)

Bei dieser Variante des Erfindungsgegenstandes, die in Fig. 1 der Zeichnung erläutert ist, ist im unteren Bereich der saugfähigen Matrixplatte eine Indikatorlinie in Form aneinandergereihter Farbindikatorflecken aufgetragen, wobei sich die Konzentration oder Zusammensetzung der Indikatorflecken von links nach rechts so verändert, daß der erste Indikatorfleck links bei weichem Wasser, der letzte Indikatorfleck rechts bei dem härtestmöglichen Wasser umschlägt und die dazwischenliegenden Indikatorflecken von links nach rechts jeweils bei zunehmender Wasserhärte in der Farbe umschlagen. Im oberen Bereich der Matrixplatte ist eine von links nach rechts schräg nach unten laufende Zeitlinie in Form einer Markierungslinie angebracht.

Bei der Verwendung dieser Vorrichtungsvariante wird die Matrixplatte zunächst in das zu entkarbonisierende oder enthärtende spezielle Wasser, wie Leitungswasser des Benutzers, eingetaucht. Dabei stellt sich in der Indikatorlinie augenblicklich eine Umschlaggrenze ein, wie im linken Teil von Fig. 1 der Zeichnung dargestellt ist, wo die vier linken Indikatorflecken von dunkel nach hell umgeschlagen sind. Da der Farbumschlag augenblicklich erfolgt, genügt kurzes Eintauchen, um diesen Verfahrensschrift abzuschließen, so daß dabei der Farbindikator ungelöst in der Matrixplatte verbleibt.

In einem zweiten Verfahrensschritt wird dann die so vorbehandelte Matrixplatte in vollentsalztes Wasser gestellt oder gehängt, das als Lösemittel für den Indikator wirkt und infolge der Kapillarität oder Saugfähigkeit der Matrixplatte alle Indikatorflecken ohne Veränderung der Umschlaggrenze in die Richtung der Zeitlinie diffundieren läßt. Wenn die nach oben diffundierende Indikatorlinie an der Stelle der Umschlaggrenze die Zeitlinie passiert, läßt sich ohne zusätzliche Denkvorgänge auf der Skala ablesen, nach wie viel Tagen das Entkarbonisierungs- oder Enthärtungsmittel erschöpft ist.

Für einen Einmaltest wählt man ein Matrixmaterial mit hoher Diffusionsgeschwindigkeit für Wasser, so daß man nach einigen Minuten ablesen kann, wie viele Stunden man bei dem speziell zu filtrierenden Wasser das Entkarbonisierungs- oder Enthärtungsmittel bis zur Erschöpfung verwenden kann. Wenn dagegen die Vorrichtung an einer Entkarbonisierungspatrone selbst angebracht werden soll, wählt man ein Matrixmaterial mit geringer Diffusionsgeschwindigkeit, um dem Benutzer direkt den Zeitpunkt der Erschöpfung anzuzeigen.

In diesem Beispiel wurde als Lösemittel vollentsalztes Wasser verwendet. Je nach der Wahl des Indikators kann auch jedes andere Lösemittel eingesetzt werden.

### Zweite Variante (Fig. 2)

Bei dieser Variante des Erfindungsgegenstandes enthält die Indikatorlinie in jedem Indikatorfleck wiederum entweder einen Farbindikator von links nach rechts steigender Konzentration oder Indikatoren unterschiedlicher chemischer Beschaffenheit, wobei wiederum der Indikator im ersten Fleck links bei weichstem Wasser und der Indikator im letzten Fleck rechts bei härtestem Wasser umschlägt. Von der ersten Variante unterscheidet sich die zweite Variante insbesondere dadurch, daß bei der zweiten Variante die Indikatoren und Lösemittel so ausgewählt und aufeinander abgestimmt sind, daß die nicht umgeschlagenen Indikatorenflecken durch das Lösemittel nicht zu der Zeitlinie hin diffundieren, sondern an der ursprünglichen Stelle der Indikatorlinie verbleiben, während die umgeschlagenen Indikatorflecken zur Zeitlinie hin diffundieren, so daß der Durchgang der Umschlaggrenze durch die Zeitlinie für den Betrachter leichter erkennbar ist. Dies ist besonders von Vorteil, wenn der Farbumschlag nicht sehr markant ist. Die als Markierungslinie vorgesehene Zeitlinie ist in der zweiten Variante entsprechend dem Diffusionsverhalten der Indikatoren in der Matrix etwas gekrümmt.

Die Verwendung der Vorrichtung gemäß der zweiten Variante entspricht der der ersten Variante. Das heißt, zunächst wird die Matrixplatte in das zu entkarbonisierende oder enthärtende Wasser eingetaucht, um den Farbumschlag in der Indikatorlinie zu bewirken, worauf dann in einem zweiten Schritt die Matrixplatte in ein Lösemittel für die umgeschlagenen Indikatorflecken gehängt oder gestellt wird.

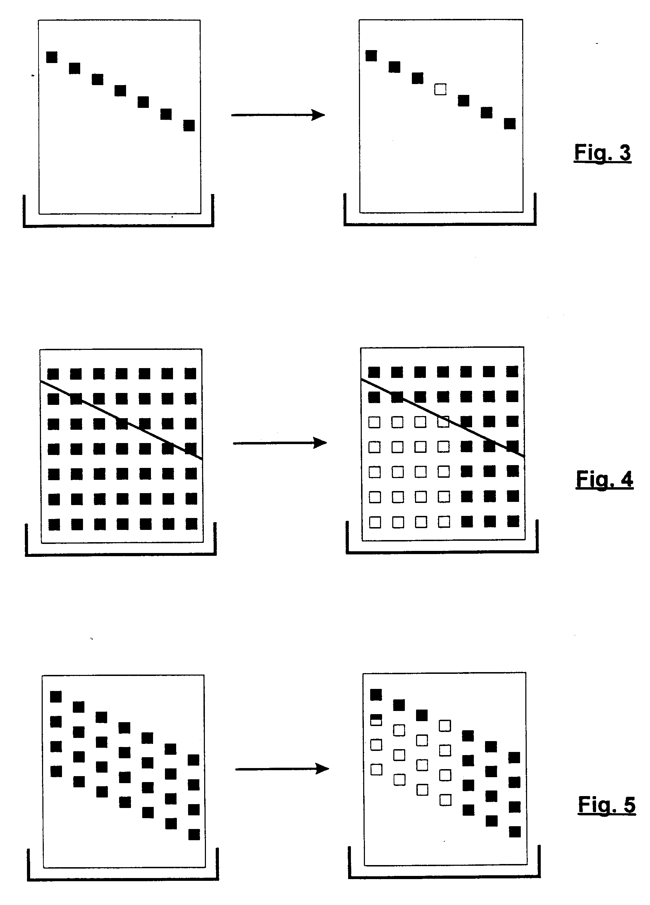

### Dritte Variante (Fig. 3)

Diese Variante des Erfindungsgegenstandes erfordert kein Lösemittel und keine Skaleneinteilung und ist dazu bestimmt, den Erschöpfungszeitpunkt unmittelbar durch Farbumschlag anzuzeigen, d. h. den Zeitpunkt, an dem das Entkarbonierungs- oder Enthärtungsmittel ausgetauscht und regeneriert werden muß. Wenn diese Variante als beschleunigter Einmaltest eingesetzt werden soll, ist aber auch bei dieser Variante entsprechend den Fig. 1 und 2 die Anbringung einer Skala erforderlich, um abzulesen, wie viele Tage das Mittel zum Entkarbonisieren oder Enthärten eines Wassers spezieller Härte eingesetzt werden kann, bevor es erschöpft ist.

Bei Verwendung der Vorrichtung gemäß der dritten Variante ist der Indikator mit von links nach rechts steigender Konzentration oder anderer Veränderung, um in jedem Indikatorfleck bei einem bestimmten Härtegrad des mit ihm in Berührung gebrachten Wassers umzuschlagen, nicht lösemittellöslich, sondern an die Matrix fest gebunden. Die von links nach rechts geneigte Indikatorlinie besteht aus einzelnen Indikatorflecken. Bei der Verwendung dieser Variante der Vorrichtung nach der Erfindung wird die Matrixplatte direkt in das zu entkarbonisierende oder zu enthärtende Wasser gestellt oder gehängt, so daß dieses Wasser durch die Saugfähigkeit und Kapillarkräfte des Matrixmaterials von der Ansaugkante, der unteren Kante der Matrixplatte, nach oben zur Indikatorlinie diffundiert. Zu dem Zeitpunkt, an dem einer der Indikatorflecken in der Indikatorlinie in der Farbe umschlägt, ist für das spezielle zu behandelnde Wasser der Erschöpfungszeitpunkt erreicht. Zu diesem Zeitpunkt muß entweder das Entkarbonisierungs- oder Enthärtungsmittel ausgetauscht oder in einem Vortest von einer vorhandenen Skala abgelesen werden, wieviel Tage das Mittel bei dem speziellen Wasser verwendet werden kann.

### Vierte Variante (Fig. 4)

Auch diese Variante arbeitet lösemittelfrei und mit matrixgebundenem Indikator. Gegenüber der dritten Variante unterscheidet sich die vierte Variante dadurch, daß die gesamte Matrixplatte mit gebundenen Indikatorflecken versehen ist, wobei die Indikatorflecken von links nach rechts stetig verändert sind, um jeweils bei unterschiedlichem Härtegrad des mit ihnen in Berührung kommenden Wassers einen Farbumschlag zu ergeben, während die Indikatorflecken in einer Reihe von unten nach oben gleiche Zusammensetzung haben. Die Indikatorlinie ist bei dieser Variante in Form einer Markierungslinie vorgesehen, die nach rechts unten abfallend durch das Muster der Indikatorflecken hindurchgelegt ist.

Bei Verwendung dieser Vorrichtung wird wiederum die Matrixplatte direkt in das zu entkarbonisierende oder zu enthärtende Wasser eingestellt oder eingehängt, so daß dieses Wasser nach oben diffundiert und dabei jeweils den Indikator bis zur Umschlaggrenze umschlagen läßt. Wenn die Umschlaggrenze die als Markierungslinie durch die matrixgebundenen Indikatorflecken gehende Indikatorlinie passiert, ist der Erschöpfungszeitpunkt erreicht bzw. läßt sich die Verwendungsdauer unmittelbar auf einer angebrachten Skala ablesen.

### Fünfte Variante (Fig. 5)

Die fünfte Variante unterscheidet sich von der vierten Variante dadurch, daß die Indikatorlinie nicht in Form einer Markierungslinie, sondern in Form der Obergrenze des aus einzelnen Indikatorflecken zusammengesetzten Indikatorfeldes vorgesehen ist. Der Erschöpfungszeitpunkt ist erreich oder ablesbar, wenn die Umschlaggrenze, d. h. der Punkt zwischen dem letzten umgeschlagenen Flecken und dem ersten nichtumgeschlagenen Flecken, die oberste Reihe der Indikatorflecken erreicht.

## Patentansprüche

1. Vorrichtung zum Anzeigen der Erschöpfung eines Entkarbonisierungs- und/oder Enthärtungsmittels für Wasser, **gekennzeichnet durch** eine saugfähige Matrix, in einer Indikatorlinie in der Matrix angeordneten lösemittellöslichen Wasserhärte-Indikator, der entlang der Indikatorlinie in derart zunehmend veränderter Weise enthalten ist, daß die Umschlaggrenze des Indikators in Abhängigkeit von der Härte eines mit dem Wasserhärte-Indikator in Berührung gebrachten Wassers an unterschiedlichen Stellen auf der Indikatorlinie liegt, und eine im Abstand von der Indikatorlinie auf der Matrix angebrachte, einer Skaleneinteilung zugeordnet Zeitlinie, wobei die Zeitlinie dem Punkt der Indikatorlinie, wo die Umschlaggrenze der größten Wasserhärte entspricht, am nächsten liegt und von dem Punkt der Indikatorlinie, wo die Umschlaggrenze der kleinsten Wasserhärte entspricht, am weitesten enfernt liegt, oder umgekehrt (Fig. 1 und 2).

2. Vorrichtung zum Anzeigen der Erschöpfung eines Entkarbonisierungs- und/oder Enthärtungsmittels für Wasser, **gekennzeichnet durch** eine saugfähige Matrix mit einer Ansaugkante, in einer Indikatorlinie in der Matrix angeordneten, matrixgebundenen Wasserhärte-Indikator, der entlang der Indikatorlinie in derart zunehmend veränderter Weise enthalten ist, daß die Umschlaggrenze des Indikators in Abhängigkeit von der Härte eines mit dem Wasserhärte-Indikator in Berührung gebrachten Wassers an unterschiedlichen Stellen auf der Indikatorlinie liegt, wobei die Ansaugkante der Matrix dem Punkt der Indikatorlinie, wo die Umschlaggrenze der größten Wasserhärte entspricht, am nächsten liegt und von dem Punkt der Indikatorlinie, wo die Umschlaggrenze der kleinsten Wasserhärte entspricht, am weitesten entfernt liegt, oder umgekehrt (Fig. 3 bis 5).

3. Vorrichtung nach Anspruch 2, **dadurch gekennzeichnet**, daß zwischen der Ansaugkante der Matrix und der Indikatorlinie weiterer Wasserhärte-Indikator in Form eines Indikatorfeldes in der Matrix angeordnet ist, wobei die Beschaffenheit des Indikators von der Ansaugkante zur Indikatorlinie im wesentlichen unverändert ist, sich aber in der Richtung senkrecht hierzu entlang dem Indikatorfeld zunehmend verändert (Fig. 4 und 5).

4. Vorrichtung nach einem der Ansprüche 1 bis 3, **dadurch gekennzeichnet**, daß die zunehmende Veränderung des Indikators in der Indikatorlinie in einer zunehmenden Konzentrationsveränderung des Indikators besteht.

5. Vorrichtung nach einem der Ansprüche 1 bis 3, **dadurch gekennzeichnet**, daß die zunehmende Veränderung des Indikators in der Indikatorlinie in einer Aufeinanderfolge unterschiedlicher Indikatoren besteht.

6. Vorrichtung nach einem der Ansprüche 1 bis 5, **dadurch gekennzeichnet**, daß sie als Indikator oder Indikatoren Farbindikatoren mit einem mit bloßem Auge sichtbaren Farbumschlag enthält.

7. Vorrichtung nach einem der Ansprüche 1 bis 6, **dadurch gekennzeichnet**, daß die Indikatorlinie und/oder Zeitlinie und/oder Ansaugkante der Matrix gerade Linien sind.

8. Vorrichtung nach einem der Ansprüche 1 bis 7, **dadurch gekennzeichnet**, daß die Indikatorlinie und/oder das Indikatorfeld aus voneinander getrennten Indikatorflecken besteht.

9. Vorrichtung nach einem der Ansprüche 1 bis 8, **dadurch gekennzeichnet**, daß die saugfähige Matrix aus einem Material mit verzögerter Diffusionsgeschwindigkeit besteht.

10. Vorrichtung nach einem der Ansprüche 1 und 4 bis 9, **dadurch gekennzeichnet**, daß der Indikator oder die Indikatoren so beschaffen sind, daß sie in einem gemeinsamen Lösemittel löslich sind und in diesem gemeinsam zur Zeitlinie diffundieren.

11. Vorrichtung nach einem der Ansprüche 1 und 4 bis 9, **dadurch gekennzeichnet**, daß der Indikator oder die Indikatoren so beschaffen sind, daß sie sich nur auf einer Seite der Umschlaggrenze in einem gemeinsamen Lösemittel auflösen und/oder in diesem allein oder bevorzugt zu der Zeitlinie hin diffundieren.

12. Verfahren zum Anzeigen der Erschöpfung eines Entkarbonisierungs- und/oder Enthärtungsmittels für Wasser unter Verwendung einer Vorrichtung nach Anspruch 1, **dadurch gekennzeichnet**, daß man zunächst die Indikatorlinie mit dem zu entkarbonisierenden und/oder zu enthärtenden Wasser in Berührung bringt, bis sich die Umschlaggrenze des Indikators in der Indikatorlinie ausgebildet hat, und sodann durch Einsaugen von Lösemittel in die saugfähige Matrix Indikator der Indikatorlinie in Lösung bringt und wenigstens bis zu der Zeitlinie diffundieren läßt.

13. Verfahren zum Anzeigen der Erschöpfung eines Entkarbonisierungs- und/oder Enthärtungsmittels für Wasser unter Verwendung einer Vorrichtung nach Anspruch 2 oder 3, **dadurch gekennzeichnet**, daß man das zu entkarbonisierende und/oder zu enthärtende Wasser an der Ansaugkante der Matrix in diese einsaugt und wenigstens bis zum Indikatorlinie diffundieren läßt.
